(19) Europäisches Patentamt
European Patent Office
Office européen des brevets

(11) Veröffentlichungsnummer: **0 313 783**
**A1**

(12) # EUROPÄISCHE PATENTANMELDUNG

(21) Anmeldenummer: **88114935.5**

(22) Anmeldetag: **13.09.88**

(51) Int. Cl.4: **C07D 249/08 , C07D 233/60 , C07D 405/06 , A61K 31/41 , A61K 31/415**

Die Bezugnahmen auf die Abbildungen 1 - 18 gelten als gestrichen (Regel 43 EPÜ).

(30) Priorität: **25.09.87 DE 3732385**

(43) Veröffentlichungstag der Anmeldung: **03.05.89 Patentblatt 89/18**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Stroech, Klaus, Dr.**
**Rolsberger Strasse 22**
**D-5650 Solingen 19(DE)**
Erfinder: **Frie, Monika, Dr.**
**Im Alten Driesch 1**
**D-5068 Odenthal(DE)**
Erfinder: **Himmler, Thomas, Dr.**
**Bonhoefferstrasse 20**
**D-5000 Köln 80(DE)**
Erfinder: **Plempel, Manfred, Dr.**
**Zwengenberger Strasse 3c**
**D-5657 Haan(DE)**

(54) **Antimykotische Mittel.**

(57) Die vorliegende Erfindung betrifft neue Hydroxyalkylazolyl-Derivate sowie deren pharmakologisch verträglichen Säureadditions-Salze zur Bekämpfung von Krankheiten, insbesondere Mykosen.

EP 0 313 783 A1

Xerox Copy Centre

## Antimykotische Mittel

Die vorliegende Erfindung betrifft neue Hydroxyalkylazolyl-Derivate und deren pharmakologisch verträgliche Säureadditions-Salze zur Behandlung von Krankheiten, insbesondere Mykosen.

Es ist bereits bekannt, daß bestimmte Azolylmethylcyclopropyl-carbinol-Derivate, wie beispielsweise 1-(4-Chlorphenyl)-1-(1-fluor-cycloprop-1-yl)-2-(1,2,4-triazol-1-yl)-ethan-1-ol und 1-(4-Chlorphenyl)-1-[1-(2,4-dichlorphenoxy)-cycloprop-1-yl]-2-(1,2,4-triazol-1-yl)-ethan-1-ol, antimykotische Eigenschaften aufweisen (vergleiche EP-OS 0 180 850). Die Wirkung dieser Stoffe ist jedoch nicht immer in allen Indikationsbereichen voll zufriedenstellend.

Es wurde gefunden, daß die neuen Hydroxyalkyl-azolyl-Derivate der Formel

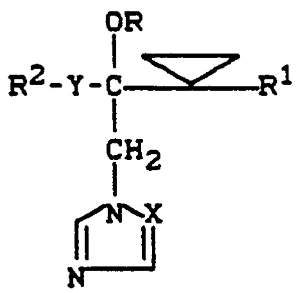

(I)

in welcher
R für Wasserstoff, Alkyl oder Acyl steht,
$R^1$ für Halogen, gegebenenfalls substituiertes Phenyl oder die Gruppierung $-Z-R^3$ steht, worin
Z für Sauerstoff, Schwefel, SO oder $SO_2$ steht und
$R^3$ für gegebenenfalls substituiertes Alkyl, gegebenenfalls substituiertes Phenyl oder gegebenenfalls substituiertes Phenylalkyl steht,
$R^2$ für gegebenenfalls substituiertes Alkyl, gegebenenfalls substituiertes Cycloalkyl, gegebenenfalls substituiertes Naphthyl, für den Rest der Formel

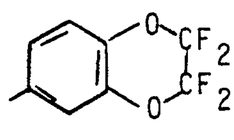

oder für den Rest der Formel

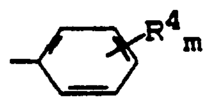

steht,

worin
$R^4$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl, für gegebenenfalls durch Alkyl mit 1 oder 2 Kohlenstoffatomen und/oder Halogen substituiertes Phenoxy, Nitro, Amino, Alkylamino, Dialkylamino, Arylamino oder Alkylcarbonylamino steht und
m für die Zahlen 0, 1, 2 oder 3 steht,
X für Stickstoff oder eine CH-Gruppe steht und
Y für die Gruppierungen $-CH_2-CH_2-$, $-CH=CH-$, $-C\equiv C-$,

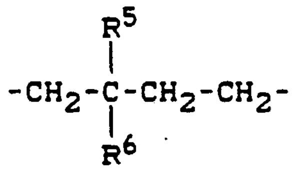 oder 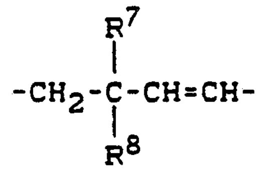 steht,

worin
$R^5$, $R^6$, $R^7$ und $R^8$ unabhängig voneinander für Wasserstoff oder Alkyl mit 1 bis 4 Kohlenstoffatomen stehen,
sowie deren Säureadditions-Salze gute antimikrobielle, insbesondere antimykotische Eigenschaften aufweisen.

Die erfindungsgemäß zu verwendenden Stoffe enthalten ein asymmetrisch substituiertes Kohlenstoffatom. Sie können daher in optischen Isomerenformen anfallen, Darüber hinaus können diejenigen Stoffe der Formel (I), in denen Y für eine -CH = CH-Gruppe oder für eine

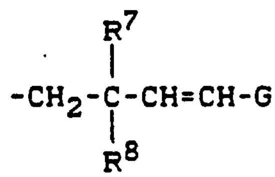Gruppe

steht, auch in Form von cis- bzw. trans-Isomeren vorliegen. Die Erfindung betrifft sowohl die einzelnen Isomeren als auch deren Gemische.

Die erfindungsgemäß zu verwendenden Hydroxyalkyl-azolyl-Derivate sind durch die Formel (I) allgemein definiert. In dieser Formel stehen vorzugsweise
R für Wasserstoff, Alkyl mit 1 bis 6 Kohlenstoffatomen oder Alkylcarbonyl mit 1 bis 6 Kohlenstoffatomen im Alkylteil,
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Z-$R^3$, worin
Z für Sauerstoff, Schwefel, SO oder $SO_2$ steht und
$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl, das gegebenenfalls im Phenylteil durch Halogen, Halogenalkyl mit 1 oder 2 Kohlenstoffatomen und 1 bis 5 Halogen atomen, Alkyl mit 1 bis 4 Kohlenstoffatomen und/oder Alkoxy mit 1 bis 4 Kohlenstoffatomen substituiert ist,
$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl, für den Rest der Formel

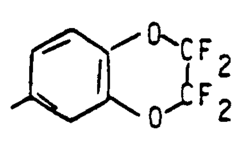

oder für den Rest der Formel

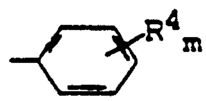 steht,

worin
$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcar bonylamino mit 1 bis 4 Kohlenstoffatomen in der Alkylgruppe steht und
m für die Zahlen 0, 1, 2 oder 3 steht,
X für Stickstoff oder eine CH-Gruppe und
Y für die Gruppierungen $-CH_2-CH_2-$, $-CH=CH-$, $-C\equiv C-$,

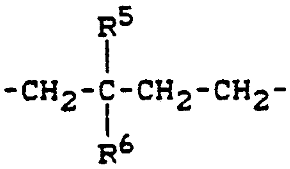 oder 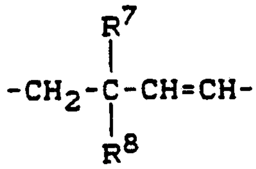 steht,

worin,
$R^5$, $R^6$, $R^7$ und $R^8$ unabhängig voneinander für Wasserstoff, Methyl oder Ethyl stehen.

Wenn m für die Zahlen 2 oder 3 steht, können die für $R^4$ stehenden Reste gleich oder verschieden sein.

Besonders bevorzugt sind diejenigen Verbindungen der Formel (I), in denen
R für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl steht,
$R^1$ für Fluor, Chlor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung der Formel $-Z-R^3$ steht, worin
Z für Sauerstoff, Schwefel, SO oder $SO_2$ steht und
$R^3$ für Methyl, Ethyl, n-Propyl, Isopropyl, n-Butyl, Isobutyl oder tert.-Butyl steht, wobei jeder dieser Reste einfach bis dreifach, gleichartig oder verschieden substituiert sein kann durch Methoxy, Ethoxy, Methylthio, Phenyl, Fluor, Chlor und/oder Brom, oder
$R^3$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder
$R^3$ für Benzyl steht, das im Phenylteil gegebenenfalls einfach bis dreifach, gleichartig oder verschieden durch Fluor, Chlor, Brom, Trifluormethyl, Methyl, Ethyl, Methoxy und/oder Ethoxy substituiert sein kann,
$R^2$ für gegebenenfalls einfach bis dreifach, gleichartig oder verschieden durch Methoxy, Ethoxy, Methylthio, Ethylthio, Phenyl, Fluor, Chlor und/oder Brom substituiertes Alkyl mit 1 bis 4 Kohlenstoffatomen steht, ferner für gegebenenfalls einfach bis dreifach, gleichartig oder verschieden durch Methyl, Ethyl, Fluor und/oder Chlor substituiertes Cycloalkyl mit 3 bis 7 Kohlenstoffatomen steht, außerdem für gegebenenfalls einfach bis dreifach, gleichartig oder verschieden durch Methyl, Fluor und/oder Chlor substituiertes Naphthyl steht oder für den Rest der Formel

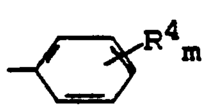 steht,

worin
$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht, oder für Nitro, Amino, Alkylamino mit 1 oder 2 Kohlenstoffatomen in der Alkylgruppe, Dialkylamino mit 1 oder 2 Kohlenstoffatomen in jeder Alkylgruppe, Phenylamino oder für Alkylcarbonylamino mit 1 oder 2 Kohlenstoffatomen in der Alkylgruppe steht,
m für die Zahlen 0, 1, 2 oder 3 steht,
X für Stickstoff oder eine CH-Gruppe steht und
Y für die Gruppierungen $-CH_2-CH_2-$, $-CH=CH-$, $-C\equiv C-$,

$$-CH_2-\overset{R^5}{\underset{R^6}{C}}-CH_2-CH_2- \quad \text{oder} \quad -CH_2-\overset{R^7}{\underset{R^8}{C}}-CH{=}CH- \quad \text{steht,}$$

worin
$R^5$, $R^6$, $R^7$ und $R^8$ unabhängig voneinander für Wasserstoff, Methyl oder Ethyl stehen.

Bevorzugte erfindungsgemäße Verbindungen sind auch pharmakologisch verträgliche Additionsprodukte aus Säuren und denjenigen Hydroxyalkyl-azolyl-Derivaten der Formel (I), in denen R, $R^1$, $R^2$, X und Y die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Als Beispiele für Hydroxyalkyl-azolyl-Derivate der Formel (I) seien die in den folgenden Tabellen aufgeführten Stoffe genannt:

**Tabelle 1**

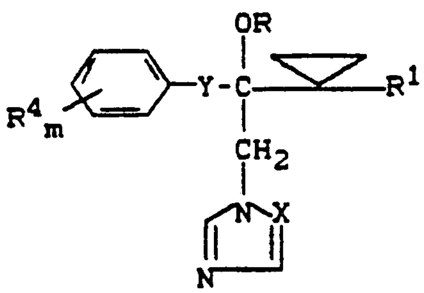

(Ic)

| $R^4_m$ | R | X | $R^1$ | Y |
|---|---|---|---|---|
| 2,4-$Cl_2$ | H | N | Cl | $-CH_2-CH_2-$ |
| 2,4-$F_2$ | H | N | Cl | " |
| 4-$CH_3$ | H | N | Cl | " |
| 4-$CF_3$ | H | N | Cl | " |
| 4-$OCF_3$ | H | N | Cl | " |
| 4-$OCH_3$ | H | N | Cl | " |
| 4-$SCH_3$ | H | N | Cl | " |
| 3-Cl | H | N | $-S-CH_3$ | " |
| 4-Cl | H | N | $-S-C_2H_5$ | " |

<u>Tabelle 1</u> (Fortsetzung)

| $R^4_m$ | R | X | $R^1$ | Y |
|---|---|---|---|---|
| 2,4,6-$Cl_3$ | " | " | Cl | $-CH_2-CH_2-$ |
| 4-Cl | " | " | F | " |
| 4-Cl | " | CH | Cl | " |
| 4-Cl | $CH_3$ | N | Cl | " |
| 4-Cl | H | " | 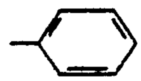 | " |
| 4-Cl | " | " | -O 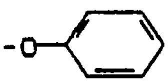 | " |
| 4-Cl | " | " | -S 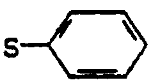 | " |
| 4-Cl | " | " | -SO 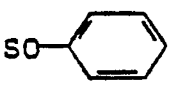 | " |
| 4-Cl | " | " | $-SO_2$ 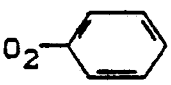 | " |
| 4- 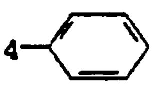 | " | " | Cl | " |
| 4-O 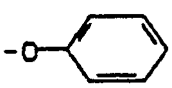 | " | " | Cl | " |
| 4-$C_4H_9$-t. | " | " | Cl | " |
| 2-Cl, 4-$CH_3$ | " | " | Cl | " |
| — | " | " | Cl | " |
| 4-Cl | $-CO-CH_3$ | " | Cl | " |
| 4-Cl | $-C_2H_5$ | " | Cl | " |
| 4-F | $CH_3$ | " | F | " |

Tabelle 1 (Fortsetzung)

| $R^4_m$ | R | X | $R^1$ | Y |
|---|---|---|---|---|
| 2,4-$Cl_2$ | H | N | Cl | -CH=CH |
| 2,4-$F_2$ | H | N | Cl | " |
| 4-$CH_3$ | H | N | Cl | " |
| 4-$CF_3$ | H | N | Cl | " |
| 4-$OCF_3$ | H | N | Cl | " |
| 4-$OCH_3$ | H | N | Cl | " |
| 4-$SCH_3$ | H | N | Cl | " |
| 3-Cl | H | N | -S-$CH_3$ | " |
| 4-Cl | H | N | -S-$C_2H_5$ | " |
| 2,4-$Cl_2$ | H | N | Cl | -C≡C- |
| 2,4-$F_2$ | H | N | Cl | " |
| 4-$CH_3$ | H | N | Cl | " |
| 4-$CF_3$ | H | N | Cl | " |
| 4-$OCF_3$ | H | N | Cl | " |
| 4-$OCH_3$ | H | N | Cl | " |
| 4-$SCH_3$ | H | N | Cl | " |
| 3-Cl | H | N | -S-$CH_3$ | " |
| 4-Cl | H | N | -S-$C_2H_5$ | " |

Tabelle 1 (Fortsetzung)

| $R^4_m$ | R | X | $R^1$ | Y |
|---|---|---|---|---|
| 2,4,6-$Cl_3$ | " | " | Cl | -CH=CH- |
| 4-Cl | " | " | F | " |
| 4-Cl | " | CH | Cl | " |
| 4-Cl | $CH_3$ | N | Cl | " |
| 4-Cl | H | " | 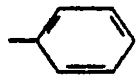 | " |
| 4-Cl | " | " | -O 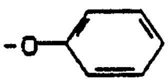 | " |
| 4-Cl | " | " | -S 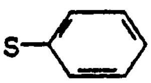 | " |
| 4-Cl | " | " | -SO 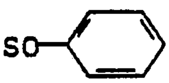 | " |
| 4-Cl | " | " | -$SO_2$ 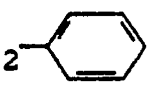 | " |
| 4 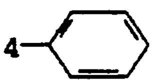 | " | " | Cl | " |
| 4-O 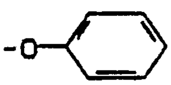 | " | " | Cl | " |
| 4-$C_4H_9$-t. | " | " | Cl | " |
| 2-Cl, 4-$CH_3$ | " | " | Cl | " |
| - | " | " | Cl | " |
| 4-Cl | -CO-$CH_3$ | " | Cl | " |
| 4-Cl | -$C_2H_5$ | " | Cl | " |
| 4-F | $CH_3$ | " | F | " |

Tabelle 1 (Fortsetzung)

| $R^4_m$ | R | X | $R^1$ | Y |
|---|---|---|---|---|
| 2,4,6-$Cl_3$ | " | " | Cl | -C≡C- |
| 4-Cl | " | " | F | " |
| 4-Cl | " | CH | Cl | " |
| 4-Cl | $CH_3$ | N | Cl | " |
| 4-Cl | H | " | 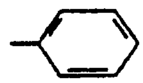 | " |
| 4-Cl | " | " | -O 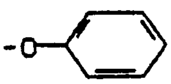 | " |
| 4-Cl | " | " | -S 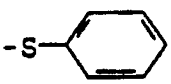 | " |
| 4-Cl | " | " | -SO 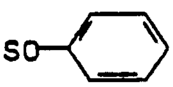 | " |
| 4-Cl | " | " | $-SO_2$ 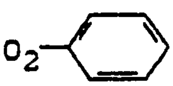 | " |
| 4- 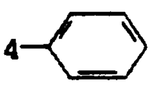 | " | " | Cl | " |
| 4-O 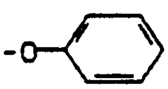 | " | " | Cl | " |
| 4-$C_4H_9$-t. | " | " | Cl | " |
| 2-Cl, 4-$CH_3$ | " | " | Cl | " |
| - | " | " | Cl | " |
| 4-Cl | $-CO-CH_3$ | " | Cl | " |
| 4-Cl | $-C_2H_5$ | " | Cl | " |
| 4-F | $CH_3$ | " | F | " |

**Tabelle 2**

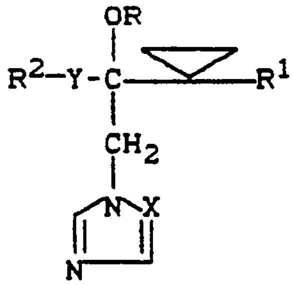 (I)

| $R^2$ | R | X | $R^1$ | Y |
|---|---|---|---|---|
| 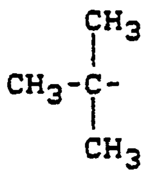 | H | N | Cl | $-CH_2-CH_2-$ |
| 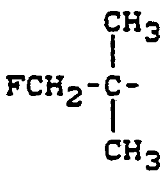 | H | N | Cl | $-CH_2-CH_2-$ |
| 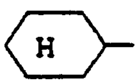 | H | N | Cl | $-CH_2-CH_2-$ |
| 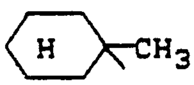 | H | N | Cl | $-CH_2-CH_2-$ |
| 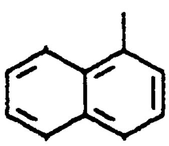 | H | N | Cl | $-CH_2-CH_2-$ |

Tabelle 2 (Fortsetzung)

| $R^2$ | R | X | $R^1$ | Y |
|---|---|---|---|---|
| 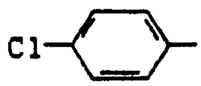 | H | N | Cl | 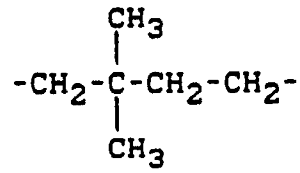 |
| 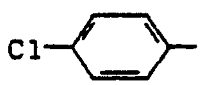 | H | N | Cl | 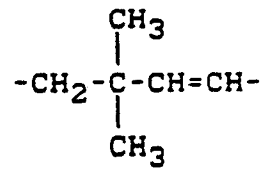 |
| 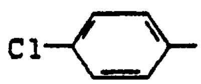 | H | N | Cl | $-CH_2-CH_2-CH=CH-$ |

Die erfindungsgemäß zu verwendenden Hydroxyalkyl-azolyl-Derivate der Formel (I) sowie ihre Säureadditions-Salze sind noch nicht bekannt. Sie sind jedoch Gegenstand einer Patentanmeldung, die noch nicht veröffentlicht ist (vergleiche die Deutsche Patentanmeldung P 3 722 794 vom 10.07.1987). Sie können nach den dort beschriebenen Verfahren erhalten werden werden, indem man

a) Oxirane der Formel

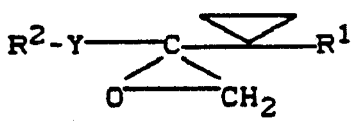 (II)

in welcher
$R^1$, $R^2$ und Y die oben angegebene Bedeutung haben,
mit Azolen der Formel

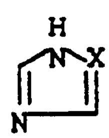 (III)

in welcher
X die oben angegebene Bedeutung hat,
in Gegenwart eines Säurebindemittels, wie beispielsweise Kaliumcarbonat oder Kalium-tert.-butylat und in Gegenwart eines Verdünnungsmittels, wie beispielsweise Acetonitril oder Dimethylformamid, bei Temperaturen zwischen 50° C und 150° C umsetzt,
oder

b) Hydroxyalkyl-azolyl-Derivate der Formel

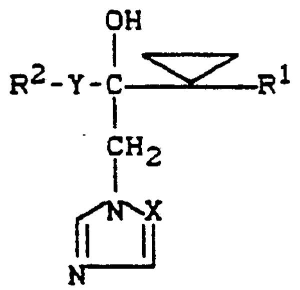

(Ia)

in welcher

R', $R^2$, X und Y die oben angegebene Bedeutung haben,

mit starken Basen, wie beispielsweise Alkalimetallamide oder -hydride in Gegenwart eines Verdünnungsmittels, wie beispielsweise Dioxan, bei Temperaturen zwischen 20° C und 100° C umsetzt und die dabei entstehenden Alkoholate der Formel

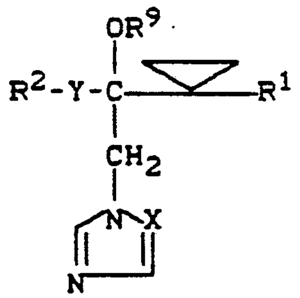

(Ib)

in welcher

R', $R^2$, X und Y die oben angegebene Bedeutung haben und

$R^9$ für einen kationischen Rest einer Base steht,

mit Halogenverbindungen der Formel

$R^{10}$-Hal (IV)

in welcher

$R^{10}$ für Alkyl oder Acyl steht und

Hal für Halogen steht,

in Gegenwart eines Verdünnungsmittels, wie beispielsweise Dioxan, bei Temperaturen zwischen 20° C und 100° C umsetzt,

und gegebenenfalls anschließend an die so erhaltenen Verbindungen der Formel (I) in üblicher Weise eine Säure addiert.

Die Oxirane der Formel (II) sind bisher noch nicht bekannt, auch sie sind Gegenstand der o.g. eigenen, älteren Deutschen Patentanmeldung, die noch nicht veröffentlicht ist. Sie lassen sich herstellen, indem man

c) Cyclopropylketone der Formel

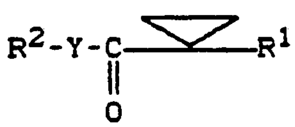

(V)

in welcher

R', $R^2$ und Y die oben angegebene Bedeutung haben,

entweder

α) mit Dimethyloxosulfonium-methylid der Formel

$$(CH_3)_2\overset{\delta\oplus}{S}O\overset{\delta\ominus}{C}H_2 \qquad (VI)$$

oder

*β*) mit Dimethylsulfonium-methylid der Formel

$$(CH_3)_2\overset{\delta\oplus}{S}\ \overset{\delta\ominus}{C}H_2 \qquad (VII)$$

in Gegenwart eines Verdünnungsmittels umsetzt, oder indem man

d) Carbinole der Formel

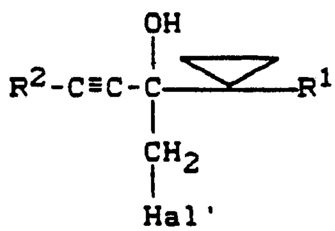

(VIII)

in welcher

$R^1$ und $R^2$ die oben angegebene Bedeutung haben und

Hal′ für Chlor oder Brom steht,

mit Basen in Gegenwart eines Verdünnungsmittels umsetzt.

Die bei der Durchführung des Verfahrens (c) als Ausgangsstoffe benötigten Cyclopropylketone der Formel (V) sind bisher noch nicht bekannt. Sie lassen sich herstellen, indem man

e) Aldehyde der Formeln

$R^2$-CHO (IX-a)

oder

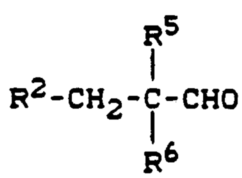

(IX-b)

in welcher

$R^2$, $R^5$ und $R^6$ die oben angegebene Bedeutung haben,

mit Methyl-cyclopropyl-ketonen der Formel

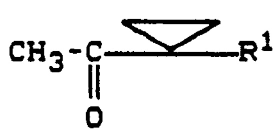

(X)

in welcher

$R^1$ die oben angegebene Bedeutung hat,

in Gegenwart eines Katalysators und in Gegenwart eines Verdünnungsmittels umsetzt und gegebenenfalls die dabei entstehenden Cyclopropylketone der Formeln

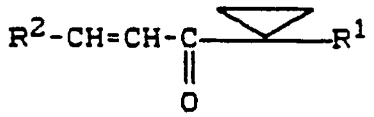

(V-a)

oder

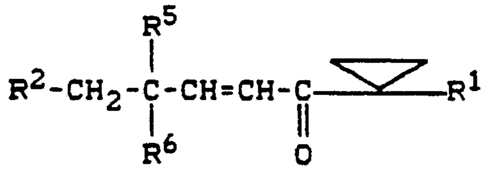

(V-b)

in welchen
$R^1$, $R^2$, $R^5$ und $R^6$ die oben angegebene Bedeutung haben,
in Gegenwart eines Katalysators und in Gegenwart eines Verdünnungsmittels hydriert,
oder indem man

f) Acetylene der Formel

$R^2$-C≡CH (XI)

in welcher
$R^2$ die oben angegebene Bedeutung hat,
mit Säurehalogeniden der Formel

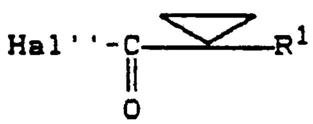

(XII)

in welcher
R' die oben angegebene Bedeutung hat und
Hal″ für Chlor oder Brom steht,
in Gegenwart eines Katalysators und in Gegenwart eines Verdünnungsmittels umsetzt.

Die bei dem Verfahren (e) als Ausgangsstoffe benötigten Aldehyde der Formeln (IX-a) und (IX-b) sind allgemein bekannte Verbindungen der organischen Chemie.

Die außerdem bei der Durchführung des Verfahrens (e) als Reaktionskomponenten benötigten Methyl-cyclopropylketone der Formel (X) sind bekannt oder lassen sich nach prinzipiell bekannten Verfahren herstellen (vgl. Synthesis 1977, 189).

Als Katalysatoren kommen bei der Durchführung der ersten Stufe des Verfahrens (e) alle für derartige Kondensationen üblichen Reaktionsbeschleuniger in Frage. Vorzugsweise verwendbar sind basische Substanzen, z.B. Alkalimetallhydroxide, wie Natriumhydroxid oder Kaliumhydroxid.

Als Verdünnungsmittel kommen bei Durchführung der ersten Stufe des Verfahrens (e) alle für derartige Umsetzungen üblichen inerten organischen Solventien in Betracht. Vorzugsweise verwendbar sind Alkohole, wie Methanol, Ethanol, Isopropanol, n-Butanol und tert.-Butanol.

Die Reaktionstemperaturen können bei der Durchführung der ersten Stufe des Verfahrens (e) innerhalb eines größeren Bereiches variiert werden. Im allgemeinen arbeitet man bei Temperaturen zwischen 0 °C und 100 °C, vorzugsweise zwischen 10 °C und 80 °C.

Die erste Stufe des Verfahrens (e) wird im allgemeinen unter Normaldruck durchgeführt. Es ist aber auch möglich, unter erhöhtem oder vermindertem Druck zu arbeiten.

Bei der Durchführung der ersten Stufe des Verfahrens (e) setzt man auf 1 Mol an Methyl-cyclopropylketon der Formel (X) 1 Mol an Aldehyd der Formel (IX) sowie eine katalytische Menge an Reaktionsbeschleuniger ein. Es ist jedoch auch möglich, die eine oder andere Komponente in einem Überschuß zu verwenden. Die Aufarbeitung erfolgt nach üblichen Methoden. Im allgemeinen geht man so vor, daß man die in festem Zustand anfallenden Reaktionsprodukte absaugt und nach gegebenenfalls vorheriger Reinigung für die weiteren Umsetzungen verwendet.

In der zweiten Stufe des Verfahrens (e) werden die Cyclopropylketone der Formeln (V-a) oder (V-b) in Gegenwart eines Katalysators und eines Verdünnungsmittels mit Wasserstoff hydriert. Man arbeitet dabei in flüssiger Phase unter Verwendung eines suspendierten, pulverförmigen Hydrierkatalysators (heterogen) oder unter Verwendung eines im Verdünnungsmittel löslichen Katalysatorkomplexes (homogen). Die Durchführung der Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung der zweiten Stufe des Verfahrens (e) inerte organische Lösungsmittel in Frage. Hierzu gehören vorzugsweise Alkohole, wie Methanol, Ethanol, Isopropanol oder Ethylenglykol; Ether, wie Diethylether, Diisopropylether, Ethylenglykolmonomethylether, Ethylenglykoldimethylether, Dioxan oder Tetrahydrofuran; gesättigte Kohlenwasserstoffe, wie n-Heptan oder Cyclohexan; aromatische Kohlenwasserstoffe, wie Toluol; sowie Ester, wie Essigsäureethylester.

Für die zweite Stufe des Verfahrens (e) geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Chloride, Oxide, Hydroxide und/oder Oxihydrate handeln.

Zusätzlich können die Metalle Kupfer, Vanadium, Molybdän, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen, diese können aber auch auf Trägermaterialien aufgebracht sein.

Als Trägermaterialien für die Wasserstoff-übertragenden Substanzen kommen beispielsweise in Frage: anorganische Materialien, wie Kieselgur, Kieselsäure, Aluminiumoxid, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien in Pulverform.

Derartige Trägerkatalysatoren können im allgemeinen 0,5 bis 50 Gew.-%, vorzugsweise 1 bis 10 Gew.-% der Wasserstoff-übertragenden Substanz, bezogen auf die Gesamtmasse des Trägerkatalysators, enthalten. Die Wasserstoff-übertagende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung der Katalysatoren, die im Verfahren (e) Verwendung finden können, kann in bekannter Weise erfolgen (siehe beispielsweise Houbel-Weyl, Methoden der organischen Chemie, Band IV, Ic, Teil I, S. 16 bis 26, Georg Thieme Verlag, Stuttgart, 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid, Nickel auf Kiesel gur, Nickel auf Aluminiumoxid sowie Nickel und Palladium auf Aluminiumoxid.

Bei Hydrierung in heterogenem System bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiven Metallen herstellbare Schwarzkatalysatoren, wie Palladium/Schwarz, Platin/Schwarz und Rhodium/Schwarz; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Bei Hydrierung im heterogenen System werden die Hydrierkatalysatoren bei der zweiten Stufe des Verfahrens (e) in einer solchen Menge eingesetzt, daß 0,05 bis 2,5, vorzugsweise 0,1 bis 1 Gew.-% Wasserstoff-übertragende Substanz bezogen auf das Gesamtgewicht des Reaktionsgemisches vorliegen.

Zur Durchführung der zweiten Stufe des Verfahrens (e) können auch Gemische aus zwei oder mehreren der genannten Hydrierkatalysatoren verwendet werden.

Die katalytische Aktivität der Hydrierkatalysatoren bleibt bei der Durchführung der zweiten Stufe des Verfahrens (e) im allgemeinen weitgehend erhalten, so daß diese bei diskontinuierlicher Arbeitsweise wiederholt eingesetzt werden können und bei kontinuierlicher Arbeitsweise längere Zeit in Gebrauch bleiben können.

Die Reaktionstemperaturen können in einem größeren Bereich variiert werden. Im allgemeinen arbeitet man im Bereich zwischen 0°C und 150°C, vorzugsweise zwischen 20°C und 120°C.

Die heterogen katalysierten Hydrierungen in der zweiten Stufe des Verfahren (e) werden vorzugsweise bei erhöhtem Druck durchgeführt. Im allgemeinen arbeitet man zwischen 1 und 150 bar, vorzugsweise zwischen 10 und 60 bar.

Außer den genannten Hydrierkatalysatoren heterogener Natur können auch homogen gelöste Hydrierkatalysatoren bei der Durchführung der zweiten Stufe des Verfahrens (e) eingesetzt werden. Die im Vergleich zu heterogenen Katalysatoren oft höhere Selektivität homogener Hydrierkatalysatoren erlaubt die selektive Hydrierung von Cyclopropylketonen der Formeln (V-a) oder (V-b), die zusätzliche hydrierbare oder hydrogenolyse-empfindliche Substituenten, wie z.B. Halogen am Phenylrest, enthalten. Solche homogenen Hydrierkatalysatoren sind beispielsweise Komplexe, die Metalle der achten Nebengruppe des Periodensystems der Elemente nach Mendelejew als Zentralatom enthalten. Bevorzugt sind dabei die Metalle Ruthenium, Rhodium, Palladium, Iridium, Kobalt und Nickel. Besonders bevorzugt sind Ruthenium, Rhodium und Iridium. Als Beispiele für derartige Metallkomplexe seien Tris-(triphenylphosphin)-rhodium(I)-chlorid, Tris-(triphenylphosphin)-ruthenium(II)-chlorid und Bis-(triphenylphosphin)-carbonyl-iridium(I)-chlorid genannt.

Bei Verwendung homogen gelöster Hydrierkatalysatoren kommen bei der Durchführung der zweiten Stufe des Verfahrens (e) als Verdünnungsmittel inerte organische Solventien in Frage. Vorzugsweise verwendbar sind Alkohole, wie Methanol, Ethanol, Isopropanol oder Ethylenglykol, ferner Kohlenwasserstoffe, wie Toluol, außerdem Ketone, wie Aceton und Butanon, und auch Ester, wie Essigsäureethylester.

Bei der Hydrierung im homogenen System werden die Hydrierkatalysatoren bei der Durchführung der zweiten Stufe des Verfahrens (e) im allgemeinen in einer solchen Menge eingesetzt, daß 0,01 bis 2,5 Mol-%, vorzugsweise 0,05 bis 1,0 Mol-% an Hydrierkatalysator-Komplex bezogen auf eingesetztes Cyclopropylketon der Formel (V-a) oder (V-b) vorhanden sind.

Die Reaktionstemperaturen können auch bei der Hydrierung im homogenen System bei der Durchführung der zweiten Stufe des Verfahrens (e) innerhalb eines größeren Bereiches variiert werden. Im allgemeinen arbeitet man bei Temperaturen zwischen 0°C und 150°C, vorzugsweise zwichen 20°C und 120°C.

Die im homogenen System durchgeführten Hydrierungen in der zweiten Stufe des Verfahrens (e) werden vorzugsweise unter erhöhtem Druck vorgenommen. Im allgemeinen arbeitet man unter Drucken zwischen 1 und 150 bar, vorzugsweise zwischen 10 und 100 bar.

In einer Variante kann die Hydrierung im homogenen System in der zweiten Stufe des Verfahrens (e) auch so durchgeführt werden, daß nicht mit molekularem Wasserstoff hydriert wird, sondern daß Reduktionsmittel eingesetzt werden, die in der Lage sind, in Gegenwart eines geeigneten Katalysators ein oder mehrere Wasserstoffatome an das Cyclopropylketon der Formel (V-a) oder (V-b) im Sinne einer Transferhydrierung zu übertragen und daher als Wasserstoffdonatoren wirken. Als Katalysatoren für eine solche Transferhydrierung kommen prinzipiell die bereits für die homogen katalysierte Hydrierung mit molekularem Wasserstoff in der zweiten Stufe des Verfahrens (e) beschriebenen Komplexe von Metallen der achten Nebengruppe des Periodensystems der Elemente nach Mendelejew in Frage.

Als Wasserstoffdonatoren kommen hierbei primäre und sekundäre, ein- oder mehrwertige Alkohole in Betracht. Vorzugsweise verwendbar sind Methanol, Ethanol,n-Propanol, i-Propanol, n-Butanol, 2-Butanol, Benzylalkohol, Ethylenglykol, 1,3-Propandiol, 1,4-Butandiol und 1,5-Pentandiol. Diese Alkohole können sowohl als Wasserstoffdonatoren als auch als Lösungsmittel dienen.

Weitere Wasserstoffdonatoren, die in der zweiten Stufe des Verfahrens (e) eingesetzt werden können, sind Alkalimetall- und Erdalkalimetall-Salze der Ameisensäure, wie Natriumformiat und Kaliumformiat, und auch die Ameisensäure selbst. - Bei Verwendung eines Salzes der Ameisensäure kann die zweite Stufe des Verfahrens (e) in Form einer Phasen-transfer-Katalyse durchgeführt werden, wobei das Cyclopropylketon der Formel (V-a) oder (V-b) und der Hydrierkatalysator in einem geeigneten inerten Solvens gelöst sind und das Formiat als wäßrige Lösung in einer zweiten Phase vorliegt. Geeignete Solventien sind deshalb in diesem Zusammenhang solche Lösungsmittel, die einerseits das Cyclopropylketon der Formel (V-a) oder (V-b) und den Hydrierkatalysator lösen, andererseits aber nicht mit Wasser mischbar sind. Derartige Lösungsmittel sind z.B. Benzol, Toluol, Chlorbenzol, Dichlorbenzole und Methylenchlorid. - Als Phasentransfer-Katalysatoren kommen alle zu diesem Zweck in der organischen Chemie einsetzbaren Reaktionsbeschleuniger in Betracht. Vorzugsweise verwendbar sind Tetrabutyl-ammonium-bromid und Methyl-tridecyl-ammoniumchlorid (Aliquat®336).

Die für die zweite Stufe des Verfahrens (e) erforderliche Reaktionszeit ist abhängig von der Reaktionstemperatur, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden. - Die Aufarbeitung erfolgt jeweils nach

üblichen Methoden.

Die bei der Durchführung des Verfahrens (f) als Ausgangsstoffe benötigten Acetylene der Formel (XI) sind bekannt oder lassen sich nach prinzipiell bekannten Verfahren in einfacher Weise herstellen.

Die bei der Durchführung des Verfahrens (f) als Reaktionskomponenten benötigten Säurehalogenide der Formel (XII) sind ebenfalls bekannt oder lassen sich nach prinzipiell bekannten Verfahren herstellen.

Als Katalysatoren kommen bei der Durchführung des Verfahrens (f) alle für derartige Umsetzungen üblichen Reaktionsbeschleuniger in Frage. Vorzugsweise verwendbar sind Kupfersalze, wie z.B. Kupferiodid.

Als Verdünnungsmittel kommen bei der Durchführung des Verfahrens (f) alle für derartige Umsetzungen üblichen inerten organischen Solventien in Betracht. Vorzugsweise verwendbar sind Ether, wie Tetrahydrofuran und Diethylether.

Die Reaktionstemperaturen können bei der Durchführung des Verfahrens (f) innerhalb eines bestimmten Bereiches variiert werden. Im allgemeinen arbeitet man bei Temperaturen zwischen -78°C und +50°C, vorzugsweise zwischen -78°C und +40°C.

Bei der Durchführung des Verfahrens (f) setzt man auf 1 Mol an Acetylen der Formel (XI) im allgemeinen 1 bis 1,2 Mol an Säurehalogenid der Formel (XII) sowie Katalysator ein. Die Aufarbeitung erfolgt nach üblichen Methoden.

Das bei dem Verfahren (c) als Reaktionskomponente benötigte Dimethyl-oxo-sulfonium-methylid der Formel (VI) ist bekannt (vgl. J. Am. Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Das bei dem Verfahren (c) außerdem als Reaktionskomponente in Betracht kommende Dimethylsulfonium-methylid der Formel (VII) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (c) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des Verfahrens (c) in einem größeren Bereich variiert werden. Im allgemeinen arbeitet man zwischen 0°C und 100°C, vorzugsweise zwischen 10°C und 60°C.

Bei der Durchführung des Verfahrens (c) setzt man auf 1 Mol an Cyclopropyl-keton der Formel (V) im allgemeinen 1 bis 3 Mol an Dimethyloxosulfonium-methylid der Formel (VI) bzw. an Dimethylsulfonium-methylid der Formel (VII) ein. Die Isolierung der Oxirane der Formel (II) erfolgt nach üblichen Methoden.

Die bei der Durchführung des Verfahrens (d) als Ausgangsstoffe benötigten Carbinole der Formel (VIII) sind bisher noch nicht bekannt. Sie lassen sich herstellen, indem man

g) Halogenketone der Formel

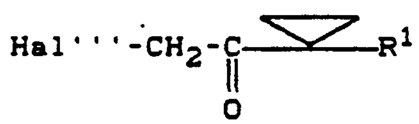

(XIII)

in welcher

$R^1$ die oben angegebene Bedeutung hat und

Hal''' für Chlor oder Brom steht,

mit Acetylensalzen der Formel

$R^2$-C≡C-Me (XIV)

in welcher

$R^2$ die oben angegebene Bedeutung hat und

Me für ein Äquivalent eines Metallkations steht,

gegebenenfalls in Gegenwart eines Säurebindemittels und in Gegenwart eines Verdünnungsmittels umsetzt.

Die bei dem Verfahren (g) als Ausgangsstoffe benötigten Halogenketone der Formel (XIII) sind teilweise bekannt (BE-PS 879 785 und DE-OS 2 944 342). Sie lassen sich herstellen, indem man

h) Methyl-cyclopropyl-ketone der Formel

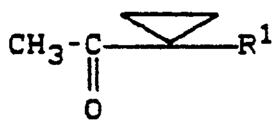

(X)

in welcher

$R^1$ die oben angegebene Bedeutung hat,

mit Chlorierungsmitteln oder Bromierungsmitteln in Gegenwart eines Verdünnungsmittels umsetzt.

Als Chlorierungsmittel und Bromierungsmittel kommen bei dem Verfahren (h) alle für derartige Umsetzungen üblichen Chlorierungs- und Bromierungsreagenzien in Betracht. Vorzugsweise verwendbar sind Sulfurylchlorid, Sulfurylbromid und Brom.

Als Verdünnungsmittel kommen bei dem Verfahren (h) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (h) innerhalb eines bestimmten Bereiches variiert werden. Im allgemeinen arbeitet man bei Temperaturen zwischen -10°C und +60°C, vorzugsweise zwischen 0°C und +40°C.

Bei der Durchführung des Verfahrens (h) arbeitet man unter Normaldruck. Es ist jedoch jeweils auch möglich, unter erhöhtem oder vermindertem Druck zu arbeiten.

Bei der Durchführung des Verfahrens (h) setzt man auf 1 Mol an Keton der Formel (X) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wäßriger Natriumhydrogencarbonat-Lösung und Wasser wäscht, dann trocknet und einengt.

Die bei dem Verfahren (g) als Reaktionskomponenten benötigten Acetylen-Salze sind durch die Formel (XIV) allgemein definiert. In dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Rest genannt wurden. Me steht vorzugsweise für einen Lithiumkation oder für ein Äquivalent eines Cer(III)-Kations.

Die Acetylen-Salze der Formel (XIV) sind bekannt oder lassen sich nach prinzipiell bekannten Verfahren herstellen.

Als Säurebindemittel kommen bei der Durchführung des Verfahrens (g) alle üblichen Säureakzeptoren in Frage.

Als Verdünnungsmittel kommen bei der Durchführung des Verfahrens (g) alle üblichen inerten organischen Solventien in Betracht. Vorzugsweise verwendbar sind aromatische Kohlenwasserstoffe, wie Toluol, und außerdem Ether, wie Diethylether, Tetrahydrofuran, tert.-Butylmethyl-ether und Gemische dieser Ether.

Die Reaktionstemperaturen können bei der Durchführung des Verfahrens (g) innerhalb eines größeren Bereiches variiert werden. Im allgemeinen arbeitet man bei Temperaturen zwischen -100°C und +100°C, vorzugsweise zwischen -80°C und +50°C.

Das Verfahren (g) wird im allgemeinen unter Normaldruck durchgeführt. Es ist aber auch möglich, unter erhöhtem oder vermindertem Druck zu arbeiten.

Bei der Durchführung des Verfahrens (g) setzt man auf 1 Mol an Halogenketon der Formel (XIII) im allgemeinen 1 bis 3 Mol an Acetylensalz der Formel (XIV) ein. Die Aufarbeitung erfolgt nach üblichen Methoden.

Als Basen kommen bei der Durchführung des Verfahrens (d) alle üblicherweise für derartige Umsetzungen geeigneten organischen und anorganischen Säurebindemittel in Betracht. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des Verfahrens (d) alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des Verfahrens (d) innerhalb eines bestimmten Bereiches variiert werden. Im allgemeinen arbeitet man bei Temperaturen zwischen -20 °C und +100 °C, vorzugsweise zwischen 0 °C und 60 °C.

Bei der Durchführung des Verfahrens (d) arbeitet man im allgemeinen unter Normaldruck. Es ist jedoch auch möglich, unter erhöhtem oder vermindertem Druck zu arbeiten.

Bei der Durchführung des Verfahrens (d) setzt man auf 1 Mol an Carbinol der Formel (VIII) im allgemeinen 1 bis 3 Mol an Base ein. Die Aufarbeitung erfolgt nach üblichen Methoden.

Die außerdem bei der Durchführung des erfindungsgemäßen Verfahrens (a) als Reaktionskomponenten benötigten Azole der Formel (III) sind allgemein bekannte Verbindungen der organischen Chemie.

Die für das erfindungsgemäße Verfahren (b) als Ausgangsstoffe benötigten Hydroxyalkylazolyl-Derivate der Formel (Ia) sind erfindungsgemäße Verbindungen. Ihre Überführung in die entsprechenden Alkoholate erfolgt in allgemein bekannter Weise, indem man sie mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^9$ in den Verbindungen der Formel (Ib) vorzugsweise für ein Alkalimetallkation, wie ein Natrium- oder Kaliumkation, oder für ein quarternäres Ammonium- oder Phosphoniumkation.

Die außerdem bei der Durchführung des erfindungsgemäßen Verfahrens (b) als Ausgangsstoffe benötigten Halogenverbindungen der Formel (IV) sind bekannt oder lassen sich nach im Prinzip bekannten Methoden herstellen.

Die Säureadditions-Salze der Verbindungen der Formel (I) können in einfacher Weise nach übliche Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelantine, Polyvinylpyrrolidon, (c) Feuchthaltemittel, z.B. Glycerin, (d) Sprengmittel z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silocone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder der Trägerstoffen.

Zur vorliegenden Erfindung gehört auch die Verwendung der erfindungsgemäßen Wirkstoffe, sowie von pharmazeutischen Zubereitungen, die einen oder mehrere erfindungs gemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in

Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

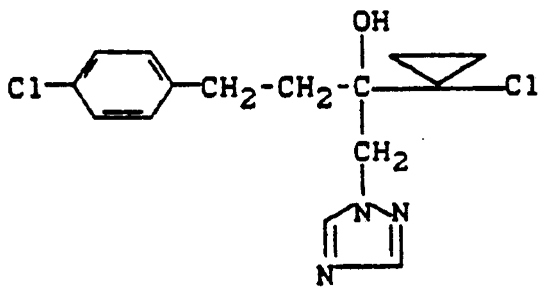

(I-1)

In ein Gemisch aus 10 g Kaliumcarbonat, 15 g (0,22 Mol) 1,2,4-Triazol und 50 ml Acetonitril wird unter Stickstoffatmosphäre eine Lösung von 13,4 g (0,05 Mol) 2-(1-Chlorcyclopropyl)-2-(4-chlorphenylethyl)-oxiran in 20 ml Acetonitril eingetropft, wobei das Reaktionsgemisch unter Rückfluß erhitzt wird. Nach beendeter Zugabe rührt man das Reaktionsgemisch noch 8 Stunden unter Rückfluß, saugt dann vom Rückstand ab und engt das Filtrat durch Abziehen des Lösungsmittels unter vermindertem Druck ein. Man nimmt den verbleibenden Rückstand in Essigester auf, wäscht die organische Phase mit Wasser, trocknet über Natriumsulfat und engt durch Abziehen des Lösungsmittels unter vermindertem Druck ein. Der anfallende Rückstand wird mit Chloroform/Ethanol = 98:2 als Laufmittel über eine Kieselgel-Säule chromatographiert. Nach Umkristallisation des dabei verbleibenden Produktes aus Cyclohexan erhält man 5,1 g (30 % der Theorie) an 2-(1-Chlorcyclopropyl)-4-(4-chlorphenyl)-1-(1,2,4-triazol-1-yl)-butan-2-ol in Form einer Festsubstanz vom Schmelzpunkt 137 °C.

Herstellung von Ausgangssubstanzen:

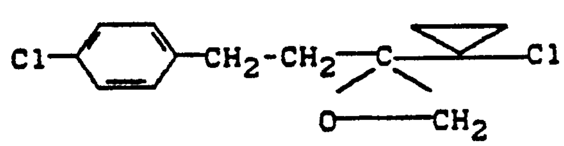

(II-1)

16 ml (0,22 Mol) Dimethylsulfid und 24,2 g (0,19 Mol) Dimethylsulfat werden in 30 ml tert.-Butanol gegeben und 14 Stunden bei 20 °C stehengelassen. Unter Rühren tropft man in das Reaktionsgemisch zunächst eine Lösung von 17 g ( 0,07 Mol) 1-Chlorcyclopropyl-4-chlorphenylethylketon in 70 ml tert.-Butanol und trägt dann 22 g Kaliumhydroxid-Pulver ein, wobei die Temperatur des Reaktionsgemisches auf 20 bis 30 °C gehalten wird. Man rührt noch 3 Stunden bei 30 °C, zieht dann das Dimethylsulfid unter vermindertem Druck ab und gießt das Reaktionsgemisch danach auf 50 ml einer 1 %igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter

vermindertem Druck eingeengt. Man erhält auf diese Weise 13,4 g (75 % der Threorie) an 2-(1-Chlorcyclopropyl)-2-(4-chlorphenylethyl)-oxiran in Form eines öligen Produktes, das ohne zusätzliche Reinigung weiter umgesetzt wird.

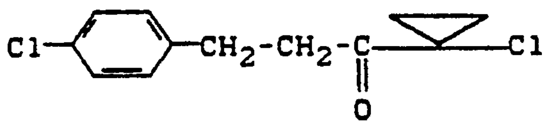

(V-1)

In einen 100 ml Autoklaven werden 460 mg (0,5 mmol) Tris-triphenylphosphin-rhodiumchlorid (= 1 Mol-%, bezogen auf die Reaktionskomponenten) gegeben. Nach dem Spülen mit Stickstoff wird eine luftfreie Lösung von 12 g (0,05 Mol) 1-Chlorcyclopropyl-4-chlorphenylethenyl-keton in 40 ml Toluol hinzugefügt und unter einem Wasserstoffdruck von 30 bar auf 50 °C erhitzt. Der Wasserstoffdruck wird zwischen 40 und 50 bar gehalten bis die Gasaufnahme beendet ist (nach etwa 1 Stunde). Anschließend läßt man 1 Stunde nachreagieren. Zur Aufarbeitung wird das Lösungsmittel unter vermindertem Druck abgezogen und der verbleibende Rückstand mit Dichlormethan als Laufmittel an Kieselgel gereinigt. Man erhält 11 g (90 % der Theorie) an 1-Chlorcyclopropyl-4-chlorphenylethyl-keton in Form eines öligen Produktes.
$^1$H-NMR (200 MHz, $CDCl_3$):
$\delta$ = 1,28-1,38 (m, 2H), 1,57-1,67 (m, 2H), 2,84 (t, 2H), 3,15 (t, 2H), 7,08-7,29 (m, 4H).

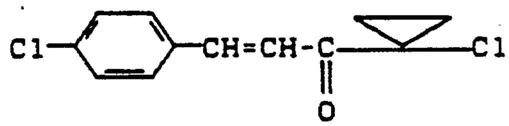

(V-2)

In ein Gemisch von 60 g (0,5 Mol) 1-Chlorcyclopropylmethyl-keton, 70 g (0,5 Mol) 4-Chlorbenzaldehyd und 250 ml Ethanol werden bei Raumtemperatur 50 ml Wasser und 10 Plätzchen festes Natriumhydroxid gegeben. Es wird 16 Stunden bei Raumtemperatur gerührt. Danach wird der ausgefallene Feststoff angesaugt. Man erhält auf diese Weise 108,5 g (89 % der Theorie) an 1-Chlorcyclopropyl-4-chlorphenylethenyl-keton in Form einer Festsubstanz vom Schmelzpunkt 92 °C.

Beispiel 2

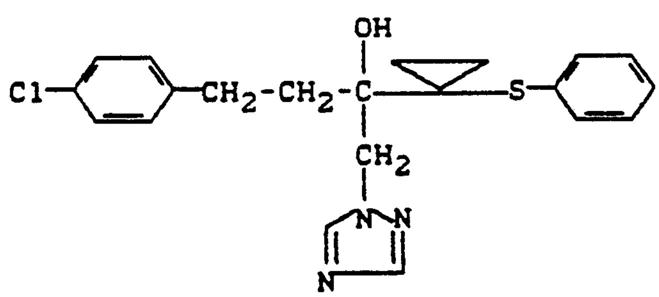

(I-2)

In ein Gemisch aus 14 g Kaliumcarbonat, 21 g (0,3 Mol) 1,2,4-Triazol und 70 ml Acetonitril wird unter Stickstoffatmosphäre eine Lösung von 25,6 g (0,1 Mol)2-(4-Chlorphenylethyl)-2-(1-phenylmercapto-cyclopropyl)-oxiran in 30 ml Acetonitril eingetropft, wobei das Reaktionsgemisch unter Rückfluß erhitzt wird. Nach beendeter Zugabe rührt man noch 8 Stunden unter Rückfluß, saugt dann vom Rückstand ab und engt das Filtrat durch Abziehen des Lösungsmittels unter vermindertem Druck ein. Man nimmt den verbleibenden Rückstand in Essigester/Toluol auf, wäscht die organische Phase mit Wasser, trocknet über Natriumsulfat und engt durch Abziehen des Lösungsmittels unter vermindertem Druck ein. Der anfallende Rückstand wird mit Chloroform/Ethanol = 99:1 als Laufmittel über eine Kieselgel-Säule chromatographiert. Nach Umkristallisation des verbleibenden Rückstandes aus Cyclohexan erhält man 11,1 g (37 % der Theorie) an 4-(4-Chlorphenyl)-2-(1-phenylmercapto-cyclopropyl)-1-(1,2,4-triazol-1-yl)-butan-2-ol in Form eines Feststoffes vom Schmelzpunkt 137 °C.

Herstellung von Ausgangssubstanzen:

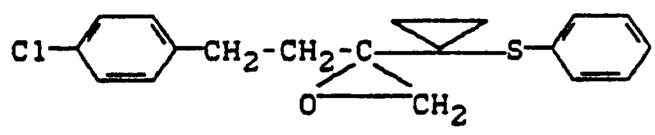

(II-2)

21 ml (0,29 Mol) Dimethylsulfid und 32,5 g (0,26 Mol) Dimethylsulfat werden in 40 ml tert.-Butanol gegeben und 14 Stunden bei Raumtemperatur stehengelassen. Unter Rühren tropft man in das Reaktionsgemisch zunächst eine Lösung von 30 g (0,095 Mol) 4-Chlorphenylethyl-1-phenylmercapto-cyclopropylketon in 90 ml tert.-Butanol und trägt dann 29,2 g Kaliumhydroxid-Pulver ein, wobei die Temperatur des Reaktionsgemisches auf 20 bis 30 °C gehalten wird. Man rührt noch 3 Stunden bei 30 °C, zieht dann das Dimethylsulfid unter vermindertem Druck ab und gießt das Reaktionsgemisch danach auf 70 ml einer 1 %igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 25,6 g (82 % der Theorie) an 2-(4-Chlorphenylethyl)-2-(1-phenylmercapto-cyclopropyl)-oxiran in Form eines öligen Produktes, das ohne zusätzliche Reinigung weiter umgesetzt wird.

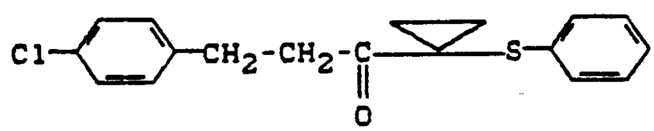

(V-3)

In einen 100 ml Autoklaven werden 460 mg (0,5 mmol) Tris-triphenylphosphin-rhodiumchlorid (= 1 Mol-%, bezogen auf die Reaktionskomponente) gegeben. Nach dem Spülen mit Stickstoff wird eine luftfreie Lösung von 15,7 g (0,05 Mol) 4-Chlorphenylethenyl-1-phenylmercaptocyclopropyl-keton in 40 ml Toluol hinzugefügt und unter einem Wasserstoffdruck von 30 bar auf 50 °C erhitzt. Der Wasserstoffdruck wird zwischen 40 und 50 bar gehalten bis die Gasaufnahme beendet ist. Anschließend läßt man 1 Stunde nachreagieren. Zur Aufarbeitung wird das Lösungsmittel unter vermindertem Druck abgezogen und der verbleibende Rückstand mit Dichlormethan als Laufmittel an Kieselgel gereinigt. Man erhält 14,2 g (90 % der Theorie) an 4-Chlorphenylethyl-1-phenylmercaptocyclopropyl-keton in Form eines öligen Produktes.
$^1$H-NMR (200 MHz, $CDCl_3$):
$\delta$ = 1,25-1,35 (m, 2H), 1,75-1,90 (m, 2H), 2,79 (t, 2H), 3,18 (t, 2H) 6,95-7,45 (m, 9H).

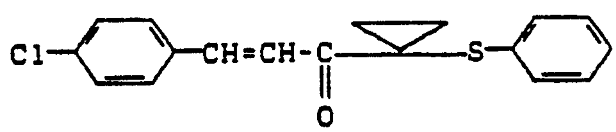

(V-4)

In ein Gemisch von 75 g (0,39 Mol) 1-Phenylmercaptocyclopropyl-methyl-keton, 56 g (0,39 Mol) 4-Chlorbenzaldehyd und 200 ml Ethanol werden bei Raumtemperatur 50 ml Wasser und 8 Plätzchen festes Natriumhydroxid gegeben. Es wird 14 Stunden bei Raumtemperatur gerührt, Danach wird der ausgefallene Feststoff abgesaugt. Man erhält auf diese Weise 120,3 g (98 % der Theorie) an 4-Chlorphenylethenyl-1-phenylmercapto-cyclopropyl-keton in Form einer Festsubstanz.
$^1$H-NMR (200 MHz, $CDCl_3$:
$\delta$ = 1,32-1,41 (m, 2H), 1,87-1,95 (m, 2H), 7,05-7,84 (m, 11H).

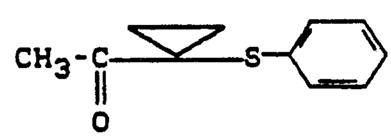

(X-1)

In eine Lösung von 100 g (0,83 Mol) 5-Chlorpentan-2-on in 400 ml Methylenchlorid wird bei 10 °C unter

Rühren eine Lösung von 134 g (0,83 Mol) Brom in 130 ml Methylenchlorid eingetropft. Das Reaktionsgemisch wird 1 Stunde bei Raumtemperatur nachgerührt, dann mit Wasser und verdünnter, wäßriger Natriumcarbonat-Lösung gewaschen und über Natriumsulfat getrocknet. Man engt das Gemisch durch Abziehen des Lösungsmittels unter vermindertem Druck ein, nimmt den Rückstand in 200 ml Methanol auf und gibt bei 5° C unter Rühren 91,5 g (0,83 Mol) Thiophenol hinzu. Danach wird ein Gemisch aus 93 g Kaliumhydroxid-Pulver in 500 ml Methanol zugetropft. Das Reaktionsgemisch wird zunächst 2 Stunden bei Raumtemperatur und dann 4 Stunden bei 40° C gerührt. Anschließend wird durch Abziehen des Lösungsmittels unter vermindertem Druck eingeengt und der verbleibende Rückstand in Methylenchlorid aufgenommen. Die organische Lösung wird nacheinander mit Wasser, verdünnter wäßriger Natronlauge und wieder mit Wasser gewaschen, danach unter vermindertem Druck eingeengt und dann einer Vakuumdestillation unterworfen. Man erhält auf diese Weise 76,8 g (48 % der Theorie) an Methyl-1-phen ylmercaptocyclopropyl-keton vom Siedepunkt 155° C/20 mbar.

Beispiel 3 und 4

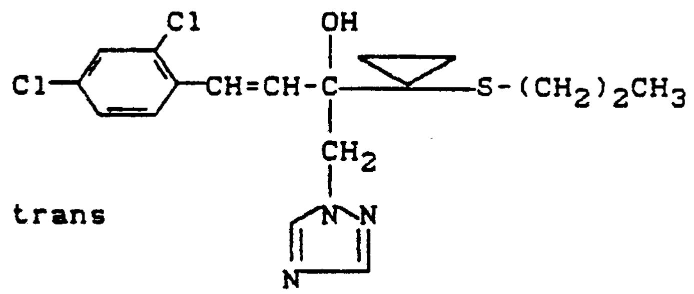

trans

(I-3)

In ein Gemisch aus 24 g (0,35 Mol) 1,2,4-Triazol, 2,7 g (0,02 Mol) Kalium-tert.-butylat und 50 ml Dimethylformamid wird bei 80° C unter Stickstoffatmosphäre und unter Rühren eine Lösung von 38,2 g (0,12 Mol) 2-(2,4-Dichlorphenylethenyl)-2-(1-propylmercaptocyclopropyl)-oxiran in 30 ml Dimethylformamid eingetropft. Man rührt 6 Stunden bei 80° C zieht dann das Lösungsmittel unter vermindertem Druck ab, nimmt den Rückstand in Essigester-Toluol auf, wäscht mit Wasser, trocknet über Natriumsulfat und engt unter vermindertem Druck ein. Der verbleibende Rückstand wird mit Chloroform als Laufmittel an Kieselgel chromatographiert. Man erhält auf diese Weise 12,1 g (26 % der Theorie) an trans-4-(2,4-Dichlorphenyl)-2-(1-propylmercaptocyclopropyl)-1-(1,2,4-triazol-1-yl)-but-3-en-2-ol in Form eines öligen Produktes.

$^1$H-NMR (200 MHz, $CDCl_3$):

$\delta$ = 0,75-1,65 (m, 9H), 2,63 (t, 2H), 4,62 (d, 1H), 4,73 (d, 1H), 6,30 (d, 1H), 6,90 (d, 1H), 7,12-7,42 (m, 3H), 7,90 (s, 1H), 8,17 (s, 1H).

Außerdem werden 1,8 g (4 % der Theorie) an cis-4-(2,4-Dichlorphenyl-2-(1-propylmercaptocyclopropyl)-1-(1,2,4-triazol-1-yl)-but-3-en-2-ol isoliert (Verbindung I-4).

$^1$H-NMR (200 MHz, $CDCl_3$):

$\delta$ = 0,75-1,10 (m, 7H), 1,50 (sex, 2H), 2,40 (t, 2H), 4,35-4,53 (m, 3H), 5,97 (d, 1H), 7,00 (d, 1H), 7,20-7,44 (m, 3H), 7,92 (s, 1H), 8,13 (s, 1H).

Herstellung von Ausgangssubstanzen:

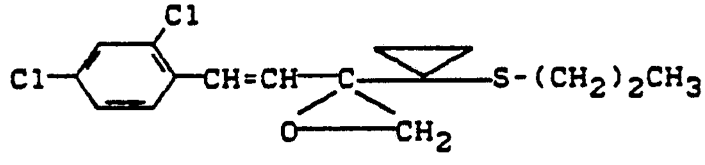

(II-3)

30 ml (0,41 Mol) Dimethylsulfid und 43,5 g (0,35 Mol) Dimethylsulfat werden in 60 ml tert.-Butanol gegeben und 14 Stunden bei Raumtemperatur stehengelassen. Unter Rühren tropft man in das Reaktionsgemisch zunächst eine Lösung von 40 g (0,13 Mol) 2,4-Dichlorphenylethenyl-1-propylmercaptocyclopropyl-keton in 120 ml tert.-Butanol und trägt dann 39,1 g Kaliumhydroxid-Pulver ein, wobei die Temperatur des Reaktionsgemisches auf 20 bis 30 °C gehalten wird. Man rührt noch 3 Stunden bei 30 °C, zieht dann das Dimethylsulfid unter vermindertem Druck ab und gießt das Reaktionsgemisch danach auf 70 ml einer 1 %igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 38,2 g (91 % der Threorie) an 2-(2,4-Dichlorphenylethenyl)-2-(1-propylmercaptocyclopropyl)-oxiran in Form eines öligen Produktes, das ohne zusätzliche Reinigung weiter umgesetzt wird.

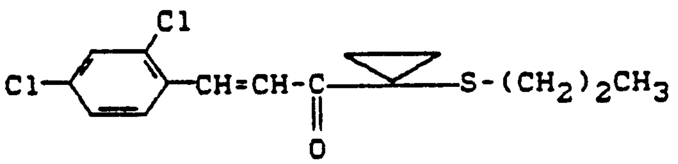

(V-5)

In ein Gemisch von 41,7 g (0,26 Mol) 1-Acetyl-1-propylmercapto-cyclopropan, 46 g (0,26 Mol) 2,4-Dichlorbenzaldehyd, 130 ml Ethanol und 30 ml Dichlormethan werden bei Raumtemperatur 5 Plätzchen festes Natriumhydroxid gegeben. Es wird 14 Stunden bei Raumtemperatur nachgerührt. Man versetzt mit 50 ml Wasser, trennt die Ölphase ab und nimmt in Dichlormethan auf. Die organische Phase wird mit Wasser gewaschen, über Natriumsulfat getrocknet und durch Abziehen des Lösungsmittels unter vermindertem Druck eingeengt. Man erhält auf diese Weise 80 g (97 % der Theorie) an 2,4-Dichlorphenylethenyl-1-propylmercaptocyclopropyl-keton (cis-trans-Isomere) in Form eines öligen Produktes.

$^1$H-NMR (200 MHz, $CDCl_3$):

$\delta$ = 0,95 (t, 3H), 1,19-1,29 (m, 2H), 1,50-1,70 (m, 4H), 2,57 (t, 2H), 7,20-7,95 (m, 5H).

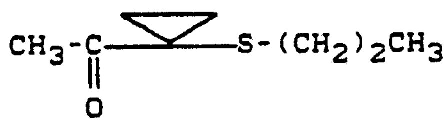

(X-2)

In eine Lösung von 100 g (0,83 Mol) 5-Chlorpentan-2-on in 400 ml Methylenchlorid wird bei 10 °C unter Rühren eine Lösung von 134 g (0,83 Mol) Brom in 130 ml Methylenchlorid eingetropft. Das Reaktionsgemisch wird 1 Stunde bei Raumtemperatur nachgerührt, dann mit Wasser und verdünnter, wäßriger Natriumcarbonat-Lösung gewaschen und über Natriumsulfat getrocknet. Man engt das Gemisch durch Abziehen des Lösungsmittels unter vermindertem Druck ein, nimmt den Rückstand in 200 ml Methanol auf und gibt bei 5 °C unter Rühren 63 g (0,83 Mol) n-Propylmercaptan hinzu. Danach wird ein Gemisch aus 93 g Kaliumhydroxid-Pulver in 500 ml Methanol zugetropft. Das Reaktionsgemisch wird zunächst 2 Stunden bei Raumtemperatur und dann 4 Stunden bei 40 °C gerührt. Anschließend wird durch Abziehen des Lösungsmittels unter vermindertem Druck eingeengt und der verbleibende Rückstand in Methylenchlorid aufgenommen. Die organische Lösung wird nacheinander mit Wasser, verdünnter wäßriger Natronlauge und wieder mit Wasser gewaschen, danach unter vermindertem Druck eingeengt und dann einer Vakuumdestillation unterworfen. Man erhält auf diese Weise 42 g (32 % der Theorie) an 1-Acetyl-1-propylmercaptocyclopropan vom Siedepunkt 107 °C/20 mbar.

Beispiel 5

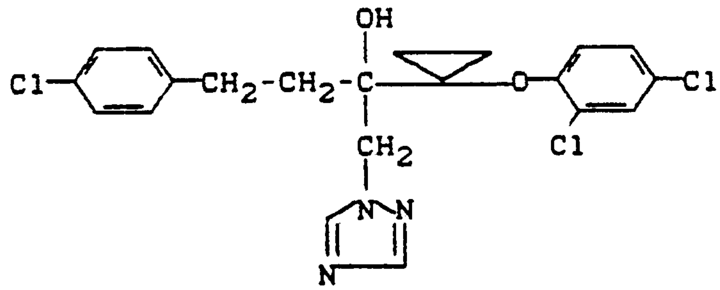

(I-5)

Nach den zuvor angegebenen Methoden wird auch 4-(4-Chlorphenyl)-2-[1-(2,4-dichlorphenoxy)-cyclopropyl]-1-(1,2,4-triazol-1-yl)-butan-2-ol in Form eines öligen Produktes erhalten.

$^1$H-NMR (200 MHz, $CDCl_3$):

$\delta$ = 0,25-0,45 (m, 2H), 0,67-0,83 (m, 1H), 0,83-1,05 (m, 1H), 1,80-2,15 (m, 2H), 2,70-2,90 (m, 1H), 2,90-3,13 (m, 1H), 4,16 (s, 1H), 4,38 (d, 1H), 4,70 (d, 1H), 7,00-7,40 (m, 7H), 8,02 (s, 1H), 8,38 (s, 1H).

Nach den Methoden, die in den zuvor angegebenen Beispielen beschrieben wurden, wurden auch die in der folgenden Tabelle 3 aufgeführten Stoffe erhalten.

## Tabelle 3

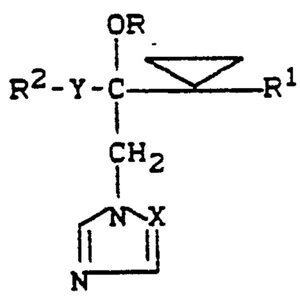

(I)

| Verbindung Nr. | $R^2$ | Y | $R^1$ | R | X | Charakterisierung durch | |
|---|---|---|---|---|---|---|---|
| I-6 | 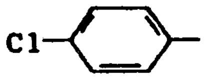 | $-CH_2-CH_2-$ | $-SCH_3$ | H | N | Fp = 115°C | |
| I-7 | 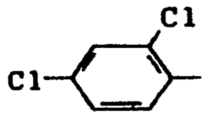 | $-CH_2-CH_2-$ | $-S(CH_2)_2CH_3$ | H | N | NMR-Spektrum | Abb. 1 |
| I-8 | 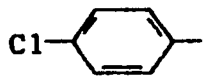 | $-CH_2-CH_2-$ | $-SCH(CH_3)_2$ | H | N | NMR-Spektrum | Abb. 2 |
| I-9 | 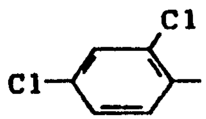 | $-CH_2-CH_2-$ | $-SC_2H_5$ | H | N | NMR-Spektrum | Abb. 3 |
| I-10 | 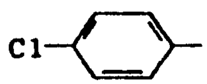 | $-CH_2-CH_2-$ | $-S-CH_2-$ 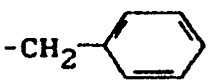 | H | N | NMR-Spektrum | Abb. 4 |
| I-11 | 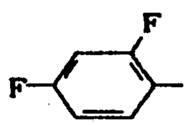 | $-CH_2-CH_2-$ | Cl | H | N | NMR-Spektrum | Abb. 5 |

| Verbindung Nr. | $R^2$ | Y | $R^1$ | R | X | Charakterisierung durch | |
|---|---|---|---|---|---|---|---|
| I-12 | 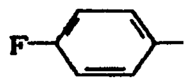 | $-CH_2-CH_2-$ | Cl | H | N | NMR-Spektrum | Abb. 6 |
| I-13 | 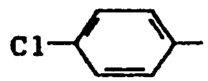 | -CH=CH- | $-SCH_3$ | H | N | NMR-Spektrum | Abb. 7 |
| I-14 | 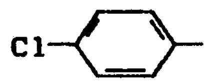 | -CH=CH-<br>(trans) | 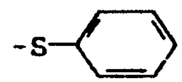 | H | N | NMR-Spektrum | Abb. 8 |
| I-15 | 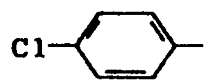 | -CH=CH-<br>(cis) | 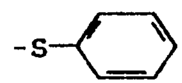 | H | N | NMR-Spektrum | Abb. 9 |
| I-16 | 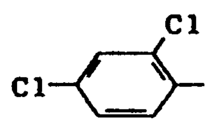 | -CH=CH- | $-SC_2H_5$ | H | N | NMR-Spektrum | Abb. 10 |
| I-17 | 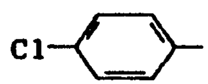 | $CH_3$<br>$-CH_2-C-CH_2-CH_2$<br>$CH_3$ | Cl | H | N | NMR-Spektrum | Abb. 11 |

| Verbindung Nr. | $R^2$ | Y | $R^1$ | R | X | Charakterisierung durch |
|---|---|---|---|---|---|---|
| I-18 | 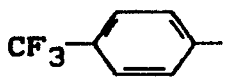 | $-CH_2-CH_2-$ | Cl | H | N | NMR-Spektrum Abb. 12 |
| I-19 | 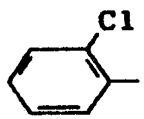 | $-CH_2-CH_2-$ | Cl | H | N | Fp: 100° C |
| I-20 | 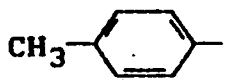 | $-CH_2-CH_2-$ | Cl | H | N | Fp: 118° C |
| I-21 | 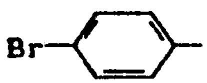 | $-CH_2-CH_2-$ | Cl | H | N | Fp: 132° C |
| I-22 | 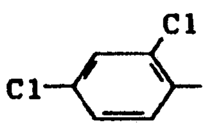 | $-CH_2-CH_2-$ | Cl | H | N | Fp: 108° C |
| I-23 | 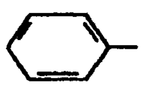 | $-CH_2-CH_2-$ | Cl | H | N | Fp: 94° C |

| Verbindung Nr. | $R^2$ | Y | $R^1$ | R | X | Charakterisierung durch | |
|---|---|---|---|---|---|---|---|
| I-24 | 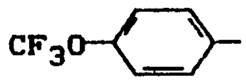 | $-CH_2-CH_2-$ | Cl | H | N | NMR-Spektrum | Abb. 13 |
| I-25 | 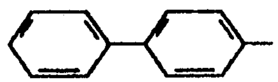 | $-CH_2-CH_2-$ | Cl | H | N | Fp: 128° C | |
| I-26 | 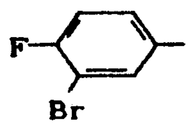 | $-CH_2-CH_2-$ | Cl | H | N | NMR-Spektrum | Abb. 14 |
| I-27 | 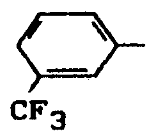 | $-CH_2-CH_2-$ | Cl | H | N | NMR-Spektrum | Abb. 15 |
| I-28 | 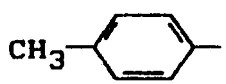 | $-CH=CH-$ | Cl | H | N | Fp: 106° C | |
| I-29 | 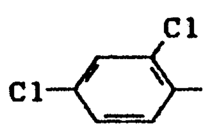 | $-CH=CH-$ | Cl | H | N | Fp: 112° C | |

| Verbindung Nr. | $R^2$ | Y | $R^1$ | R | X | Charakterisierung durch |
|---|---|---|---|---|---|---|
| I-30 | 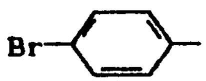 | -CH=CH- | Cl | H | N | Fp: 130° C |
| I-31 | 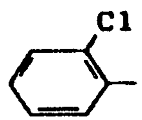 | -CH=CH- | Cl | H | N | Fp: 133° C |
| I-32 | 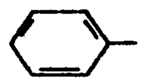 | -CH=CH- | Cl | H | N | Fp: 90° C |
| I-33 | 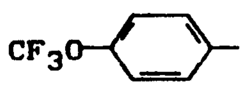 | -CH=CH- | Cl | H | N | Fp: 67° C |
| I-34 | 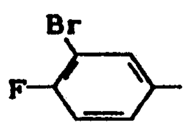 | -CH=CH- | Cl | H | N | Fp: 126° C |
| I-35 | 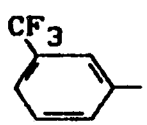 | -CH=CH- | Cl | H | N | NMR-Spektrum Abb. 16 |

| Verbindung Nr. | $R^2$ | Y | $R^1$ | R | X | Charakterisierung durch |
|---|---|---|---|---|---|---|
| I-36 | 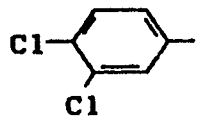 | -CH=CH- | Cl | H | N | NMR -Spektrum Abb. 17 |
| I-37 | 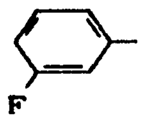 | -CH=CH- | Cl | H | N | NMR-Spektrum Abb. 18 |
| I-38 | $(CH_3)_3C$- | -CH=CH- | Cl | H | N | Fp: 74° C |
| I-39 | 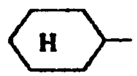 | -CH=CH- | Cl | H | N | Fp: 95° C |
| I-40 | 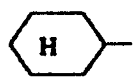 | $-CH_2-CH_2-$ | Cl | H | N | Fp: 67° C |
| I-41 | 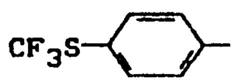 | -CH=CH- | Cl | H | N | $R_f$:0,32 ($CH_2Cl_2/CH_3COOC_2H_5$ 4:1) |
| I-42 | 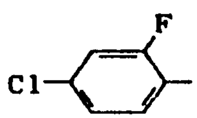 | -CH=CH- | Cl | H | N | Fp:92° C |

ɪL1,0

| Verbindung Nr. | $R^2$ | Y | $R^1$ | R | X | Charakterisierung durch |
|---|---|---|---|---|---|---|
| I-43 | 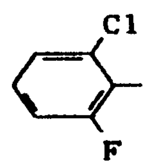 | -CH=CH- | Cl | H | N | Fp:112° C |
| I-44 | 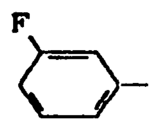 | $-CH_2-CH_2-$ | Cl | H | N | $R_f$: 0,34 ($CH_2Cl_2/CH_3COOC_2H_5$ 4:1) |
| I-45 | 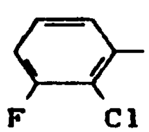 | -CH=CH- | Cl | H | N | Fp: 112° C |

| Verbin-dung Nr. | $R^2$ | Y | $R^1$ | R | X | Charakterisierung durch |
|---|---|---|---|---|---|---|
| I-46 | 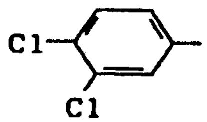 | $-CH=CH-$ | Cl | H | N | Fp: 137° C |
| I-47 | 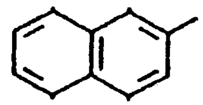 | $-CH=CH-$ | Cl | H | N | Fp: 114-116° C |
| I-48 | 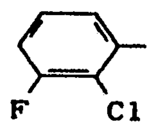 | $-CH_2-CH_2-$ | Cl | H | N | Fp: 84° C |
| I-49 | 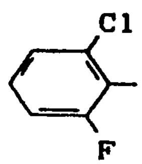 | $-CH_2-CH_2-$ | Cl | H | N | Fp: 72° C |
| I-50 | 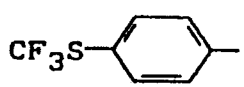 | $-CH_2-CH_2-$ | Cl | H | N | Fp: 88° C |

| Verbin-dung Nr. | $R^2$ | Y | $R^1$ | R | X | Charakterisierung durch |
|---|---|---|---|---|---|---|
| I-51 | 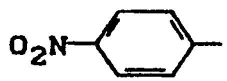 | $-CH_2-CH_2-$ | Cl | H | N | Fp: 105° C |
| I-52 | 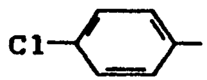 | -CH=CH- | Cl | H | N | Fp: 122° C |
| I-53 | 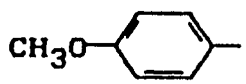 | $-CH_2-CH_2-$ | Cl | H | N | Fp: 90° C |
| I-54 | 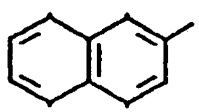 | $-CH_2-CH_2-$ | Cl | H | N | Fp: 134° C |
| I-55 | 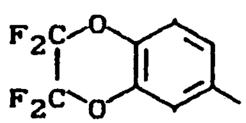 | -CH=CH- | Cl | H | N | Öl |
| I-56 | 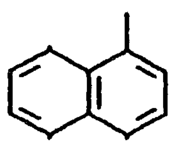 | $-CH_2-CH_2-$ | Cl | H | N | Fp: 102° C |

| Verbin-dung Nr. | $R^2$ | Y | $R^1$ | R | X | Charakterisierung durch |
|---|---|---|---|---|---|---|
| I-57 | 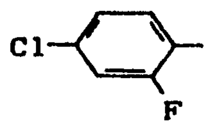 | $-CH_2-CH_2-$ | Cl | H | N | Fp: 112° C |
| I-58 | 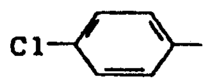 | -C≡C- | Cl | H | N | Fp: 112° C |
| I-59 | 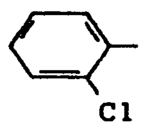 | -C≡C- | Cl | H | N | Öl |
| I-60 | 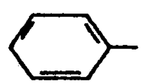 | -C≡C- | Cl | H | N | Fp: 116-118° C |
| I-61 | 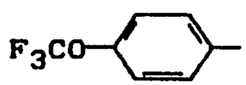 | $-CH_2-CH_2-$ | 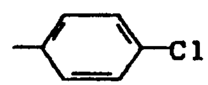 | H | N | Fp: 132° C |

| Verbindung Nr. | $R^2$ | Y | $R^1$ | R | X | Charakterisierung durch |
|---|---|---|---|---|---|---|
| I-62 | 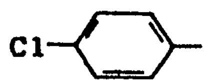 | $-CH_2-CH_2-$ | 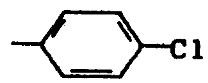 | H | N | Fp: 123° C |
| I-63 | 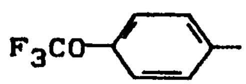 | -CH=CH- | 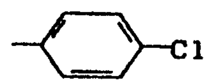 | H | N | Fp: 82° C |
| I-64 | 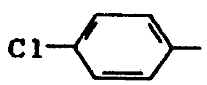 | -CH=CH- | 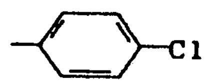 | H | N | Fp: 146° C |

Verwendungsbeispiele

In dem folgenden Beispielen werden die nachstehend angegebenen Verbindungen als Vergleichssubstanzen eingesetzt:

(A) 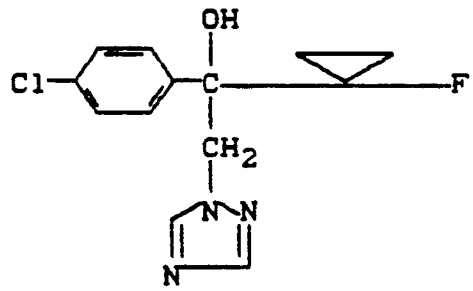

(B) 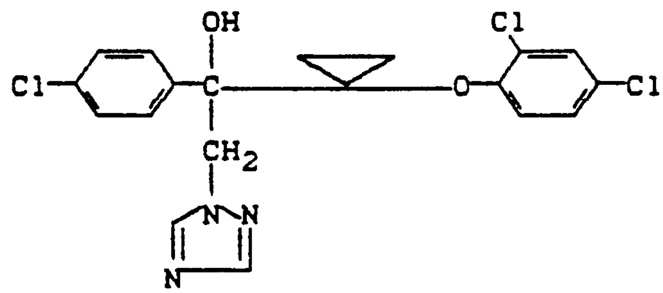

**(bekannt aus EP-OS 0 180 850)**

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze:

Sabouraud's milieu d'epreuve

b) für Hefen:

Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigten z.B. die erfindungsgemäßen Verbindungen I-1, I-3, I-6, I-8, I-9, I-10, I-12, I-13, I-16, I-17, I-19, I-20, I-22, I-23, I-24, I-25, I-28, I-29, I-30, I-31, I-32, I-33, I-34, I-38 und I-39 eine bessere antimykotische Wirksamkeit als die aus dem Stand der Technik bekannten Verbindungen (A) und (B).

Tabelle A:

Antimykotische in-vitro-Wirksamkeit

MHK-Werte in µg/ml Nährmedium

| Wirkstoff | Tricho-phyton mentagr. | Micro-sporum canis | Candida albicans | Toru-opsis gla-brata | Asper-gillus fumi-gatus |
|---|---|---|---|---|---|
| (A)(bekannt) | >64 | - | >64 | - | >64 |
| (B)(bekannt) | 64 | - | 16 | 64 | >64 |
| Verbindungen gemäß Herstellungsbeispiel | | | | | |
| I-1 | <1 | <1 | 2 | <1 | <1 |
| I-3 | 8 | - | 4 | 4 | 16 |
| I-6 | <1 | 2 | 4 | 16 | <1 |
| I-8 | 2 | - | 8 | 32 | 4 |
| I-9 | 8 | - | 4 | 16 | 16 |
| I-10 | <1 | 8 | 2 | 4 | 64 |
| I-12 | <1 | 4 | 8 | 32 | <1 |
| I-13 | <1 | 4 | 2 | 2 | <1 |
| I-16 | <1 | <1 | 8 | <1 | 4 |
| I-17 | 2 | - | <1 | 2 | 4 |
| I-19 | <1 | 2 | 4 | 16 | 2 |
| I-20 | <1 | 2 | 4 | 2 | <1 |
| I-22 | <1 | 4 | 2 | 2 | <1 |
| I-23 | <1 | 2 | 4 | 8 | <1 |

Tabelle A:

Antimykotische in-vitro-Wirksamkeit

| Wirkstoff | MHK-Werte in µg/ml Nährmedium Tricho-phyton mentagr. | Micro-sporum canis | Candida albi-cans | Toru-opsis gla-brata | Asper-gillus fumi-gatus |
|---|---|---|---|---|---|
| Verbindungen gemäß Herstellungsbeispiel | | | | | |
| I-24 | <1 | 4 | 16 | 4 | <1 |
| I-25 | <1 | 2 | <1 | 2 | <1 |
| I-28 | <1 | <1 | <1 | <1 | <1 |
| I-29 | <1 | <1 | 16 | 8 | <1 |
| I-30 | <1 | <1 | 2 | 4 | <1 |
| I-31 | <1 | 4 | 8 | 8 | 2 |
| I-32 | <1 | 4 | 4 | 8 | 2 |
| I-33 | <1 | <1 | 8 | <1 | <1 |
| I-34 | 2 | - | 4 | 4 | <1 |
| I-38 | <1 | 4 | 4 | 4 | 8 |
| I-39 | 2 | - | 16 | 16 | 2 |

Beispiel B

Antimykotische in-vivo-Wirksamkeit (oral) bei Mäusecandidose

Versuchsbeschreibung :

Mäuse vom Typ SPF-$CF_1$ wurden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10 - 100 mg/kg Körpergewicht der Präparate oral behandelt.

Ergebnis:

Unbehandelte Tiere starben 3 bis 6 Tage post infektionem. Die Überlebensrate am6.Tag post infektionem betrug bei unbehandelten Kontrolltieren etwa 5 %.

In diesem Test zeigen z.B. die erfindungsgemäßen Verbindungen (I-1), (I-6), (I-13), (I-29, (I-30), (I-33)

und (I-34) eine bessere Wirkung als die aus dem Stand der Technik bekannten Verbindungen (A) und (B).

Tabelle B

Antimykotische in-vivo-Wirkung (oral) bei Mäuse-Candidose

| Wirkstoff | Wirkung |
|---|---|
| (A)(bekannt) | k.W. |
| (B)(bekannt) | k.W. |
| Verbindung gemäß Herstellungsbeispiel | |
| (I-1) | +++ |
| (I-6) | +++ |
| (I-13) | +++++ |
| (I-29) | +++ |
| (I-30) | +++++ |
| (I-33) | +++++ |
| (I-34) | +++ |

Zeichenerklärung:

| | | |
|---|---|---|
| +++++ | = sehr gute Wirkung | = 90 % Überlebende am 6.Tag.p.i. |
| ++++ | = gute Wirkung | = 80 % Überlebende am 6.Tag.p.i. |
| +++ | = Wirkung | = 60 % Überlebende am 6.Tag.p.i. |
| ++ | = schwache Wirkung | = 40 % Überlebende am 6.Tag.p.i. |
| + | = Spur Wirkung | = unter 40 % Überlebende am 6. Tag p.i. |
| k.W. | | = kein Unterschied zur unbehandelten Infektionskontrolle |

Beispiel C / Formulierungen

1.) Lösung:

| | | |
|---|---|---|
| Wirkstoff gemäß Formel (I): | | 10 g |
| Alkohol, rein (96 %ig) | : | 300 g |
| Isopropylmyristat | : | 526 g |
| | | 836 g |

2.) Creme:

| | | |
|---|---|---|
| Wirkstoff gemäß Formel (I): | | 10 g |
| Aralcel 60 (Sorbitan-monostearat) | : | 20 g |
| Tween 60 (Polyoxyethylen (2) -sorbitan-monostearat) | : | 15 g |
| Walrat, künstlich (Mischung vom Estern von gesättigten Fettsäuren $C_{14}$-$C_{18}$ und Fettalkoholen $C_{14}$-$C_{18}$) | : | 30 g |
| Lanette O | : | 100 g |
| Eutanol G (2-Octyl-dodecanol) | : | 135 g |
| Benzylalkohol | : | 10 g |
| Wasser, entmineralisiert | : | 680 g |
| | | 1000 g |

## Ansprüche

1. Hydroxyalkyl-azolyl-Derivate der Formel

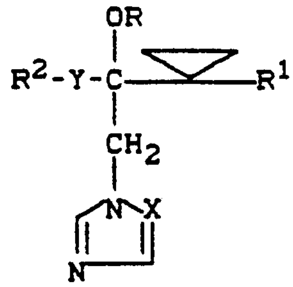

in welcher
R für Wasserstoff, Alkyl oder Acyl steht,
$R^1$ für Halogen, gegebenenfalls substituiertes Phenyl oder die Gruppierung -Z-$R^3$ steht,
worin
Z für Sauerstoff, Schwefel, SO oder $SO_2$ steht und
$R^3$ für gegebenenfalls substituiertes Alkyl, gegebenenfalls substituiertes Phenyl oder gegebenenfalls substituiertes Phenylalkyl steht,
$R^2$ für gegebenenfalls substituiertes Alkyl, gegebenenfalls substituiertes Cycloalkyl, gegebenenfalls substituiertes Naphthyl, für den Rest der Formel

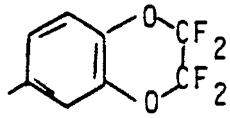

oder für den Rest der Formel

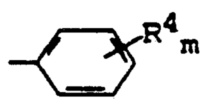 steht,

worin
$R^4$ für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und oder Halogen substituiertes Phenyl, für gegebenenfalls durch Alkyl mit 1 oder 2 Kohlenstoffatomen und oder Halogen substituiertes Phenoxy, Nitro, Amino, Alkylamino, Dialkylamino, Arylamino oder Alkylcarbonylamino steht und
m für die Zahlen 0, 1, 2 oder 3 steht,
X für Stickstoff oder eine CH-Gruppe steht und
Y für die Gruppierungen $-CH_2-CH_2-$, $-CH=CH-$, $-C\equiv C-$,

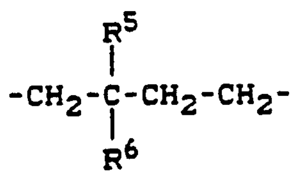 oder 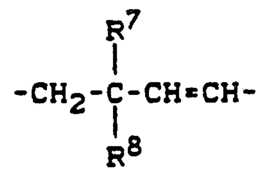 steht,

worin
$R^5$, $R^6$, $R^7$ und $R^8$ unabhängig voneinander für Wasserstoff oder Alkyl mit 1 bis 4 Kohlenstoffatomen

stehen,
sowie deren Säureadditions-Salze zur Bekämpfung von Krankheiten.

2. Hydroxyalkyl-azolyl-Derivate nach Anspruch 1
in denen
R für Wasserstoff, Alkyl mit 1 bis 6 Kohlenstoffatomen oder Alkylcarbonyl mit 1 bis 6 Kohlenstoffatomen im Alkylteil,
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Z-$R^3$, worin
Z für Sauerstoff, Schwefel, SO oder $SO_2$ steht und
$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,
$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl, für den Rest der Formel

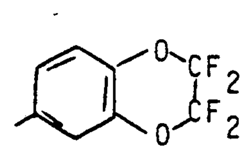

oder für den Rest der Formel

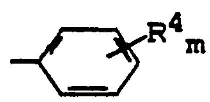 steht,

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und
m für die Zahlen 0, 1, 2 oder 3 steht,
X für Stickstoff oder eine CH-Gruppe und
Y für die Gruppierungen $-CH_2-CH_2-$, $-CH=CH-$, $-C{\equiv}C-$,

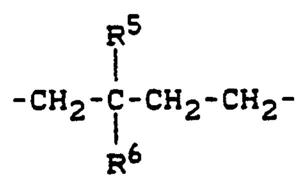 oder 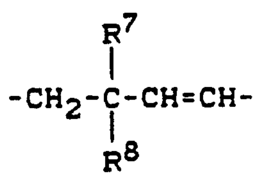 steht,

worin
$R^5$, $R^6$, $R^7$ und $R^8$ unabhängig voneinander für Wasserstoff, Methyl oder Ethyl stehen,
sowie deren Säureadditions-Salze zur Bekämpfung von Krankheiten.

3. Hydroxyalkyl-azolyl-Derivate nach Anspruch 1
in denen
R für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl steht,
$R^1$ für Fluor, Chlor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung der Formel -Z-$R^3$ steht, worin
Z für Sauerstoff, Schwefel, SO oder $SO_2$ steht und
$R^3$ für Methyl, Ethyl, n-Propyl, Isopropyl, n-Butyl, Isobutyl oder tert.-Butyl steht, wobei jeder dieser Reste einfach bis dreifach, gleichartig oder verschieden substituiert sein kann durch Methoxy, Ethoxy, Methylthio, Phenyl, Fluor, Chlor und/oder Brom, oder
$R^3$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder
$R^3$ für Benzyl steht, das im Phenylteil gegebenenfalls einfach bis dreifach, gleichartig oder verschieden durch Fluor, Chlor, Brom, Trifluormethyl, Methyl, Ethyl, Methoxy und/oder Ethoxy substituiert sein kann,
$R^2$ für gegebenenfalls einfach bis dreifach, gleichartig oder verschieden durch Methoxy, Ethoxy, Methylthio, Ethylthio, Phenyl, Fluor, Chlor und/oder Brom substituiertes Alkyl mit 1 bis 4 Kohlenstoffatomen steht, ferner für gegebenenfalls einfach bis dreifach, gleichartig oder verschieden durch Methyl, Ethyl, Fluor und/oder Chlor substituiertes Cycloalkyl mit 3 bis 7 Kohlenstoffatomen steht, außerdem für gegebenenfalls einfach bis dreifach, gleichartig oder verschieden durch Methyl, Fluor und/oder Chlor substituiertes Naphthyl steht oder für den Rest der Formel

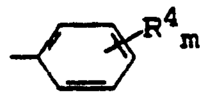 steht,

worin
$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht, oder für Nitro, Amino, Alkylamino mit 1 oder 2 Kohlenstoffatomen in der Alkylgruppe, Dialkylamino mit 1 oder 2 Kohlenstoffatomen in jeder Alkylgruppe, Phenylamino oder für Alkylcarbonylamino mit 1 oder 2 Kohlenstoffatomen in der Alkylgruppe steht,
m für die Zahlen 0, 1, 2 oder 3 steht,
X für Stickstoff oder eine CH-Gruppe steht und
Y für die Gruppierungen $-CH_2-CH_2-$, $-CH=CH-$, $-C\equiv C-$,

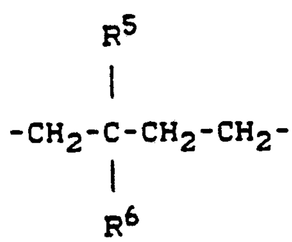 oder 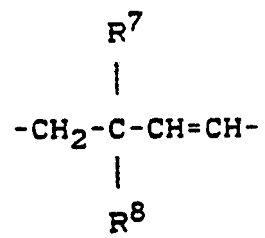 steht,

worin
$R^5$, $R^6$, $R^7$ und $R^8$ unabhängig voneinander für Wasserstoff, Methyl oder Ethyl stehen,
sowie deren Säureadditions-Salze zur Bekämpfung von Krankheiten.

4. 2-(1-Chlorcyclopropyl)-4-(4-chlorphenyl)-1-(1,2,4-triazol-1-yl)-butan-2-ol

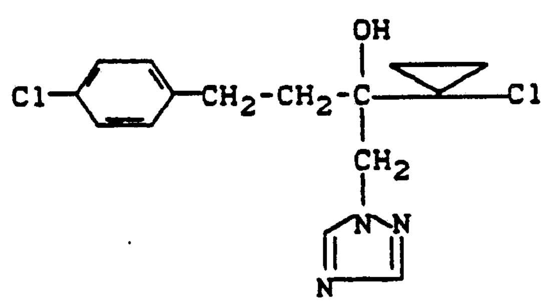

sowie dessen Säureadditions-Salze zur Bekämpfung von Krankheiten.

5. 4-(4-Chlorphenyl)-2-(1-phenylmercapto-cyclopropyl)-1-(1,2,4-triazol-1-yl)-butan-2-ol

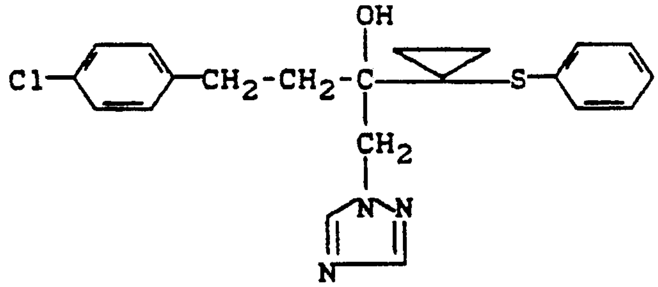

sowie dessen Säureadditions-Salze zur Bekämpfung von Krankheiten.

6. 4-(2,4-Dichlorphenyl)-2-(1-propylmercaptocyclopropyl)-1-(1,2,4-triazol-1-yl)-but-3-en-2-ol

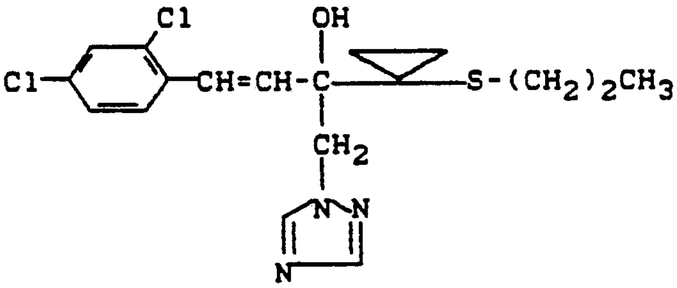

sowie dessen Säureadditions-Salze zur Bekämpfung von Krankheiten.

7. 4-(4-Chlorphenyl-2-[1-(2,4-dichlorphenoxy)-cyclopropyl]-1-(1,2,4-triazol-1-yl)-butan-2-ol

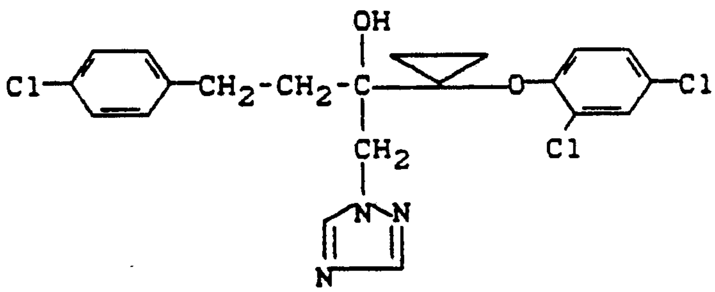

sowie dessen Säureadditions-Salze zur Bekämpfung von Krankheiten.

8. Hydroxyalkylazolyl-Derivate nach Ansprüchen 1-7 zur Bekämpfung von Mykosen.

9. Arzneimittel, enthaltend Hydroxalkylazolyl-Derivate nach Ansprüchen 1-7.

10. Antimykotische Mittel enthaltend Hydroxyalkylazolyl-Derivate nach Ansprüchen 1-7.

11. Verwendung der Hydroxyalkylazolyl-Derivate nach Ansprüchen 1-7 bei der Herstellung von Arzneimitteln zur Bekämpfung von Krankheiten.

12. Verwendung der Hydroxyalkylazolyl-Derivate nach Ansprüchen 1-7 bei der Herstellung von Arzneimitteln zur Bekämpfung von Mykosen.

Europäisches Patentamt

# EUROPÄISCHER RECHERCHENBERICHT

Nummer der Anmeldung

EP 88 11 4935

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 040 345 (BAYER AG)<br>* Seiten 1-3 * | 1-12 | C 07 D 249/08<br>C 07 D 233/60<br>C 07 D 405/06<br>A 61 K 31/41<br>A 61 K 31/415 |
| Y | EP-A-0 143 379 (BAYER AG)<br>* Seiten 1-3 * | 1-12 | |
| D,Y | EP-A-0 180 850 (BAYER AG)<br>* Seiten 1-3 * | 1-12 | |

RECHERCHIERTE SACHGEBIETE (Int. Cl.4)

C 07 D 249/00
C 07 D 233/00
C 07 D 405/00
A 61 K 31/00

Der vorliegende Recherchenbericht wurde für alle Patentansprüche erstellt

| Recherchenort | Abschlußdatum der Recherche | Prüfer |
|---|---|---|
| DEN HAAG | 28-11-1988 | BRIGHENTI |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)